# EUROPEAN PATENT APPLICATION

(11) **EP 4 838 226 A1**
(43) Date of publication of application: **11.11.2026**
(21) Application number: 24915680.3
(22) Date of filing: 29.11.2024
(51) Int. Cl.: G01N 33/92, G01N 33/52

(54) **METHOD FOR QUANTIFYING PLASMALOGENS AND KIT FOR QUANTIFYING PLASMALOGENS**

(30) Priority: 05.01.2024 JP 2024000431
(71) Applicant: Institute of Rheological Function of Food Co., Ltd., Kasuya-gun, Fukuoka 811-2501 (JP); Kyushu University, National University Corporation, Nishi-ku Fukuoka-shi Fukuoka 819-0395 (JP)
(72) Inventor: FUJINO, Takehiko, Fukuoka-shi, Fukuoka 813-0043 (JP); MAWATARI, Shiro, Fukuoka-shi, Fukuoka 813-0016 (JP); HONSHO, Masanori, Fukuoka-shi, Fukuoka 819-0395 (JP)
(74) Representative: V.O.
(86) International application number: PCT/JP2024/042293
(87) International publication number: WO 2025/146757

(57) **Abstract**

A method of quantifying plasmalogen includes: a measurement sample preparation step of mixing a specimen containing protein and plasmalogen with Schiff reagent, to obtain a measurement sample; a control sample preparation step of mixing the specimen with a solvent of the Schiff reagent, to obtain a control sample; a measurement step of measuring a degree of a first optical property of the measurement sample after reaction of the Schiff reagent with an aldehyde generated from a vinyl ether bond of the plasmalogen, and a degree of an optical property of the control sample; and a determination step of determining a plasmalogen concentration in the specimen by comparing a corrected degree obtained by correcting the degree of the first optical property with the degree of the optical property of the control sample, with a degree of a second optical property of a standard sample containing Schiff reagent and plasmalogen having a known concentration, the degree of the second optical property being obtained after reaction of the Schiff reagent with an aldehyde generated from a vinyl ether bond of the plasmalogen.

## Description

### Technical Field

The present disclosure relates to a method of quantifying plasmalogen, and a kit for quantifying plasmalogen.

### Background Art

Plasmalogen is a type of phospholipid having an antioxidant action, and is a glycerophospholipid. Plasmalogen is present in all mammalian tissues, and accounts for about 18% of phospholipids in the human body. Since plasmalogen is mostly bound to polyunsaturated fatty acids such as docosahexaenoic acid and arachidonic acid, plasmalogen is involved in the storage of polyunsaturated fatty acids, and the release of secondary messengers of intercellular signals such as prostaglandin and leukotriene produced from these polyunsaturated fatty acids. Plasmalogen is also involved in, for example, cell fusion and ion transport. Since the vinyl ether bond (alkenyl bond) of plasmalogen is particularly sensitive to oxidative stress, plasmalogen also has an antioxidant function for cells. Plasmalogen exhibits a protective action against oxidative stress in the body. For example, Non Patent Literature 1 and 2 reported that the plasmalogen concentration in plasma serves as a biomarker for arteriosclerosis, cancer, and the like.

Plasmalogen is known to be decreased in diseases of the brain and nervous system, such as dementia, Parkinson's disease, depression, and schizophrenia. For example, Non Patent Literature 3 reported that ethanolamine plasmalogen is significantly decreased in the frontal lobe and the hippocampus in the brain in Alzheimer's disease. Non Patent Literature 4 discloses that the plasmalogen concentration in blood is decreased in patients with Alzheimer's disease.

As the relationship between plasmalogen and diseases has become more evident, there has been a demand for a technique that enables simple measurement of plasmalogen in vivo. The vinyl ether bond of plasmalogen is known to react specifically and quantitatively with iodine in a methanol solution. As shown in Non Patent Literature 5, plasmalogen can be quantified by measuring a decrease in the absorption of light having a wavelength of 355 nm by iodine. The method using iodine disclosed in Non Patent Literature 5 is relatively simple, but has the disadvantage that detection sensitivity is low.

The vinyl ether bond of plasmalogen is unstable to acids, and plasmalogen is decomposed by an acid into an aliphatic aldehyde and a lysophospholipid. The aliphatic aldehyde can usually be measured as a dimethyl acetal using gas chromatography-mass spectrometry. On the other hand, the lysophospholipid can be measured by two-dimensional thin-layer chromatography or the like.

Patent Literature 1 discloses, as a method of quantifying ethanolamine plasmalogen, a method of quantifying plasmalogen including a step of treating ethanolamine lysoplasmalogen with phospholipase D, the ethanolamine lysoplasmalogen being generated by hydrolyzing ethanolamine plasmalogen in a specimen with phospholipase A1.

Patent Literature 2 discloses a method of quantifying choline plasmalogen using phospholipase D, which acts on choline plasmalogen to hydrolyze the phosphate ester at the sn-3 position of the choline plasmalogen, thereby causing the release of choline.

Further, use of liquid chromatography-mass spectrometry (LC-MS) has enabled accurate identification of lipids in a mixture and identification of plasmalogen by detecting fragment ions characteristic to the head group class and a fatty acid or an alcohol esterified to the glycerol backbone.

Non Patent Literature 6 qualitatively analyzes the presence or absence of plasmalogen in *Clostridium perfringens* by a color reaction between an aliphatic aldehyde generated by acid treatment of plasmalogen and Schiff reagent (fuchsin-aldehyde reagent).

### Citation List

### Patent Literature

Patent Literature 1: Unexamined Japanese Patent Application Publication No. 2016-111929
Patent Literature 2: Unexamined Japanese Patent Application Publication No. 2014-082991

### Non Patent Literature

Non Patent Literature 1: Megumi Nishimukai and 2 other authors, "Absorptive characteristics of plasmalogen and availability of serum/plasma plasmalogen as the biomarker", Oleoscience, 2015, 15(2), 53-60
Non Patent Literature 2: Marcia Cristina Fernandes Messias and 3 other authors, "Plasmalogen lipids: functional mechanism and their involvement in gastrointestinal cancer", Lipids in Health and Disease, 2018, 17, 41-53
Non Patent Literature 3: Zhizhong Guan and 5 other authors, "Decrease and structural modifications of phosphatidylethanolamine plasmalogen in the brain with Alzheimer disease", J Neuropathol Exp Neurol, 1999, 58(7), 740-747
Non Patent Literature 4: Dayan B. Goodenowe and 17 other authors, "Peripheral ethanolamine plasmalogen deficiency: a logical causative factor in Alzheimer's disease and dementia" 2007, J Lipid Res, 48(11), 2485-2498
Non Patent Literature 5: Eugene L. Gottfried and Maurice M. Rapport, "The biochemistry of plasmalogens. I. Isolation and characterization of phosphatidal choline, a pure native plasmalogen", 1962, J Biol Chem, 237, 329-333
Non Patent Literature 6: David R. Jackson and 7 other authors, "Plasmalogen biosynthesis by anaerobic bacteria: identification of a two-gene operon responsible for plasmalogen production in Clostridium perfringens", 2021, ACS Chem Biol, 16, 6-13

### Summary of Invention

### Technical Problem

Any of the methods disclosed in Non Patent Literature 5 and Patent Literature 1 and 2 requires an extraction process of a lipid fraction using an organic solvent. In addition, since the methods require time and expensive analytical instruments for analysis, measurement of a large number of specimens is difficult. Although the method using Schiff reagent in Non Patent Literature 6 enables qualitative detection of plasmalogen in cells, the method cannot be used for quantitative analysis.

The present disclosure was made in view of the above circumstances and aims to provide a method of quantifying plasmalogen and a kit for quantifying plasmalogen, the method and the kit being capable of simply and rapidly quantifying plasmalogen without an operation of extracting a lipid fraction.

### Solution to Problem

Even when Schiff reagent was used for quantification of plasmalogen in a specimen containing protein such as plasma, no proportional relationship was observed between the volume of the specimen, that is, the amount of plasmalogen, and the degree of color development, resulting in failure to quantify plasmalogen. The present inventors intensively studied to discover conditions for quantifying plasmalogen in a cell-free specimen by a color reaction of Schiff reagent, thereby completing the present disclosure.

A method of quantifying plasmalogen according to a first aspect of the present disclosure includes:
a measurement sample preparation step of mixing a specimen containing protein and plasmalogen with Schiff reagent, to obtain a measurement sample;
a control sample preparation step of mixing the specimen with a solvent of the Schiff reagent, to obtain a control sample;
a measurement step of measuring a degree of a first optical property of the measurement sample after reaction of the Schiff reagent with an aldehyde generated from a vinyl ether bond of the plasmalogen, and a degree of an optical property of the control sample; and
a determination step of determining a plasmalogen concentration in the specimen by comparing a corrected degree obtained by correcting the degree of the first optical property with the degree of the optical property of the control sample, with a degree of a second optical property of a standard sample containing Schiff reagent and plasmalogen having a known concentration, the degree of the second optical property being obtained after reaction of the Schiff reagent with an aldehyde generated from a vinyl ether bond of the plasmalogen.

The standard sample may be a plurality of standard samples containing the plasmalogen at concentrations different from each other and the Schiff reagent, and
in the determination step, the plasmalogen concentration in the specimen may be determined based on a calibration curve prepared from the degrees of the second optical property of the plurality of standard samples after the reaction, and on the corrected degree.

The degree of the first optical property, the degree of the optical property of the control sample, and the degree of the second optical property may be measured as fluorescence intensities.

The optical properties may be color development.

The degree of the first optical property, the degree of the optical property of the control sample, and the degree of the second optical property may be measured as absorbances.

The plasmalogen having a known concentration may be dissolved in ethanol.

The specimen may be cell-free.

The solvent of the Schiff reagent may be a sulfurous acid solution.

A kit for quantifying plasmalogen according to a second aspect of the present disclosure includes:
Schiff reagent; and
a solvent of the Schiff reagent.

### Advantageous Effects of Invention

According to the present disclosure, plasmalogen can be simply and rapidly quantified without an operation of extracting a lipid fraction.

### Brief Description of Drawings

FIG. 1 is a diagram illustrating the relationship between the corrected absorbance and the plasma volume in the sample according to Test Example 1;
FIG. 2A is a diagram illustrating the method of separation of plasmalogen used for the preparation of the calibration curve according to Example 1, by high-performance liquid chromatography (HPLC);
FIG. 2B is a diagram illustrating a chromatogram of the lipid fraction extracted by HPLC in Example 1;
FIG. 2C is a diagram illustrating a chromatogram of the ethanolamine plasmalogen (PL-PE) in Example 1;
FIG. 3 is a diagram illustrating the calibration curve according to Example 1;
FIG. 4 is a diagram illustrating the plasmalogen concentration according to measurement of the fluorescence intensity in Example 2; and
FIG. 5 is a diagram illustrating the plasmalogen concentration according to measurement of the absorbance in Example 2.

### Description of Embodiments

Embodiments according to the present disclosure are described below with reference to drawings. The present disclosure is not limited to the following embodiments and drawings. In the following embodiments, expressions such as "have", "include", or "contain" also encompass the meaning of "consist of" or "be constituted by".

### Embodiments

The method of quantifying plasmalogen according to the present embodiment includes a measurement sample preparation step, a control sample preparation step, a measurement step, and a determination step. In the measurement sample preparation step, a specimen and Schiff reagent are mixed to obtain a measurement sample. Since the quantification method according to the present embodiment quantifies plasmalogen in a specimen containing protein, the specimen contains the protein and the plasmalogen. It should be noted that there are also cases where the specimen does not contain plasmalogen, and in such cases, the quantification method determines that plasmalogen is not contained in the specimen, or is not more than a detection limit.

More specifically, in the measurement sample preparation step, Schiff reagent and the specimen described above are mixed under acidic conditions. For example, in cases where the solvent of the Schiff reagent is acidic, the Schiff reagent and the specimen can be mixed under acidic conditions. More specifically, the pH of the mixture obtained by mixing the Schiff reagent and the specimen may be less than 7. The pH is preferably less than 5, less than 4, less than 3, or less than 2.

The specimen is not particularly limited, and is a body fluid derived from a living body, a cell culture liquid, or the like. Examples of the specimen include blood, plasma, serum, urine, sweat, cerebrospinal fluid, bile, pancreatic juice, amniotic fluid, and ascites. Preferably, the specimen is cell-free. Preferably, the specimen is plasma. The living body is a human or a non-human animal, preferably a mammal. Examples of the mammal include primates such as chimpanzees; laboratory animals such as rats, mice, and rabbits; domestic animals such as pigs, cows, horses, sheep, and goats; and companion animals such as dogs and cats.

Schiff reagent is a reagent that develops color specifically with an aldehyde. The phrase "develops color specifically with an aldehyde" means that the reagent does not react with a ketone but reacts with an aldehyde to develop color. Since Schiff reagent does not react with a ketone, Schiff reagent reacts specifically with plasmalogen even in cases where the specimen contains protein. As the Schiff reagent, a commercially available product may be used, or the Schiff reagent may be prepared by dissolving basic fuchsin in hot water, cooling the resulting solution, adding anhydrous sodium sulfite and concentrated hydrochloric acid thereto, and then appropriately diluting the resulting mixture with water.

The mixing ratio between the specimen and the Schiff reagent, mixed together in the measurement sample preparation step, is not particularly limited. For example, the volume ratio between the specimen and the Schiff reagent (specimen: Schiff reagent) is 1:5 to 1:50, 1:5 to 1:40, 1:5 to 1:30, or 1:5 to 1:20.

In the control sample preparation step, the specimen and a solvent of the Schiff reagent are mixed to obtain a control sample. Regarding the solvent of the Schiff reagent, in cases where the solvent of the Schiff reagent used in the measurement sample preparation step is a sulfurous acid solution, the specimen and the sulfurous acid solution are mixed in the control sample preparation step. In cases where the solvent of the Schiff reagent used in the measurement sample preparation step is water containing anhydrous sodium sulfite and concentrated hydrochloric acid, the specimen and the water containing anhydrous sodium sulfite and concentrated hydrochloric acid are mixed in the control sample preparation step. The mixing ratio between the specimen and the solvent mixed in the control sample preparation step is preferably the same as the mixing ratio between the specimen and the Schiff reagent in the measurement sample preparation step.

In the measurement step, a degree of an optical property of the measurement sample after reaction of Schiff reagent with an aldehyde generated from the vinyl ether bond of the plasmalogen, and a degree of an optical property of the control sample, are measured. In order to allow the aldehyde and the Schiff reagent to react with each other, for example, the measurement sample may be incubated at room temperature, more preferably at 37°C. The reaction time is, for example, 5 to 60 minutes, 10 to 50 minutes, 20 to 40 minutes, or 25 to 35 minutes.

The optical properties are color development, coloring, light absorption, luminescence, fluorescence, or the like. In cases where color development is measured as the optical properties, in the measurement step, the degree of color development A of the measurement sample after reaction of Schiff reagent with an aldehyde generated from the vinyl ether bond of the plasmalogen, and the degree of color development B of the control sample, are measured. As described above, since the vinyl ether bond of plasmalogen is unstable to acids, plasmalogen is decomposed under acidic conditions to generate an aldehyde. In the measurement step, the degree of color development A caused by reaction of the generated aldehyde with Schiff reagent is measured. In the measurement step, first, the degree of color development A may be measured and then the degree of color development B may be measured; the degree of color development B may be measured and then the degree of color development A may be measured; or the degree of color development A and the degree of color development B may be measured simultaneously.

The degree of color development A and the degree of color development B can also be visually measured, but are preferably measured as absorbances using a spectrophotometer. The absorbances can be measured using a known method and a known instrument. In the measurement of the absorbances, for example, the mixed solution described above is irradiated with light having an absorption wavelength suitable for the Schiff reagent described above, and the absorptivity is measured. The wavelength of the irradiation light is 500 to 600 nm, 520 to 580 nm, or 540 to 560 nm, preferably 550 nm.

Since fuchsin contained in Schiff reagent has a structure containing a plurality of benzene rings, a product generated by reaction of an aldehyde with Schiff reagent has fluorescence. For this reason, fluorescence may be measured as the optical properties. In such a case, for example, the degree of the optical property of the measurement sample and the degree of the optical property of the control sample are measured as fluorescence intensities. The fluorescence intensities can be measured using a known method and a known instrument. In cases where fluorescence intensities are measured as the optical properties, in the measurement step, the fluorescence intensity of the measurement sample obtained after reaction of Schiff reagent with an aldehyde generated from the vinyl ether bond of the plasmalogen, and the fluorescence intensity of the control sample, are measured. Also in the fluorescence measurement, the fluorescence intensity of the measurement sample after the reaction may be measured and then the fluorescence intensity of the control sample may be measured; the fluorescence intensity of the control sample may be measured and then the fluorescence intensity of the measurement sample after the reaction may be measured; or the fluorescence intensity of the measurement sample after the reaction and the fluorescence intensity of the control sample may be measured simultaneously.

In the determination step, the plasmalogen concentration in the specimen is determined by comparing a corrected degree obtained by correcting the degree of the optical property of the measurement sample with the degree of the optical property of the control sample, with a degree of an optical property of a standard sample containing Schiff reagent and plasmalogen having a known concentration, the degree of the optical property of the standard sample being obtained after reaction of the Schiff reagent with an aldehyde generated from the vinyl ether bond of the plasmalogen.

The determination step is described below in more detail, taking as an example a case where color development is measured as the optical properties. In the determination step, the plasmalogen concentration in the specimen is determined by comparing a corrected degree obtained by correcting the degree of color development A with the degree of color development B of the control sample, with the degree of color development C (second color development) of the standard sample after the reaction. In cases where the degree of color development A and the degree of color development B are measured as absorbances, the corrected degree is, for example, the value (V₁) obtained by subtracting the value of the absorbance of the control sample from the value of the absorbance of the measurement sample. The plasmalogen having a known concentration is, for example, plasmalogen dissolved in ethanol. The plasmalogen may be plasmalogen extracted from an animal tissue or the like by a known method, or may be commercially available plasmalogen.

In cases where the degree of color development A and the degree of color development B are measured as absorbances, the degree of color development C is also measured as an absorbance. For example, the degree of color development C is the absorbance of a standard sample prepared by mixing Schiff reagent and plasmalogen at concentration C₁. Here, in cases where the above-described value V₁ is the same as the absorbance of the standard sample, the plasmalogen concentration in the specimen is determined to be C₁.

In cases where absorbances are measured as the degrees of the optical properties, the optical property of the standard sample after the reaction is also measured as the fluorescence intensity. For example, in cases where the corrected degree (the value (V₂) obtained by subtracting the value of the fluorescence intensity of the control sample from the value of the fluorescence intensity of the measurement sample) is the same as a value of the fluorescence intensity of a standard sample obtained by mixing Schiff reagent and plasmalogen at concentration C₂ and allowing the reaction to proceed, the plasmalogen concentration in the specimen is determined to be C₂.

In the determination step, the plasmalogen concentration in the specimen may be determined based on a calibration curve and the above corrected degree. The calibration curve is prepared using a plurality of standard samples containing plasmalogen at concentrations different from each other and Schiff reagent, based on the degrees of the optical property of the plurality of standard samples after the reaction. For example, the concentrations of plasmalogen in the standard samples may be set within a range that includes a plasmalogen concentration expected in the specimen. The concentrations, different from each other, of plasmalogen in the standard samples are, for example, 3, 4, 5, 6, 7, or not less than 10 different concentrations. The concentration range of plasmalogen in the standard samples is, for example, 0.005 to 0.1 µg/µL.

In cases where the degrees of the optical property are absorbances, specifically, the plasmalogen concentration in the specimen can be determined by substituting the value V₁ obtained as the corrected degree into the regression equation of the obtained calibration curve. Further, in cases where the degrees of the optical property are fluorescence intensities, the plasmalogen concentration in the specimen can be determined by substituting the value V₂ obtained as the corrected degree into the regression equation of the obtained calibration curve.

According to the method of quantifying plasmalogen according to the present embodiment, the concentration of plasmalogen in a specimen can be determined based on the degree of the optical property after reaction of Schiff reagent with plasmalogen having a known concentration in a standard sample. Therefore, plasmalogen can be simply and rapidly quantified without an operation of extracting a lipid fraction or the like. Further, in the method of quantifying plasmalogen according to the present embodiment, the concentration of plasmalogen in a specimen may be determined based on the calibration curve and the corrected degree. As illustrated in Example 1 below, the correlation coefficient of the calibration curve obtained by the quantification method is favorable, so that the concentration of plasmalogen in a specimen can be determined with high accuracy.

In the method of quantifying plasmalogen according to the present embodiment, the degrees of color development A, color development B, and color development C as optical properties may be measured as absorbances. The absorbances can be simply measured with a relatively inexpensive apparatus, and the measurement can be easily carried out for a large number of specimens.

Further, in the method of quantifying plasmalogen according to the present embodiment, the optical properties of the measurement sample, the control sample, and the standard sample may be measured as absorbances. Fluorescence is preferred from the viewpoint of the facts that simple measurement is possible, that higher detection sensitivity can be achieved compared to absorbance, and that plasmalogen at lower concentration can be quantified.

In another aspect according to the present embodiment, a kit for quantifying plasmalogen is provided. The kit for quantifying plasmalogen includes Schiff reagent and a solvent of the Schiff reagent. As described above, the Schiff reagent is used for the preparation of the measurement sample and the standard sample. As described above, the solvent is used for the preparation of the control sample. The kit for quantifying plasmalogen may further include plasmalogen that can be used for the preparation of the control sample. In another aspect according to the present embodiment, a reagent for quantifying plasmalogen, the reagent including a solvent of the Schiff reagent, is provided.

In another aspect of the present embodiment, use of a solvent of the Schiff reagent for the production of a kit for quantifying plasmalogen, or for the production of a reagent for quantifying plasmalogen, is provided. In another aspect of the present embodiment, a solvent of the Schiff reagent for use in the quantification of plasmalogen is provided.

The present disclosure is described more specifically by way of the following Examples, but the present disclosure is not limited by the Examples.

### Examples

### [Test Example 1: Study of Color Reaction between Human Plasma and Schiff Reagent]

Venous blood of a subject was collected into a blood collection tube containing heparin (manufactured by Terumo Corporation), centrifuged at 1000×g for 5 minutes, and plasma as the supernatant was collected and cryopreserved. To 10, 15, or 20 µL of plasma, 190, 185, or 180 µL of Schiff reagent (pH<2.0, manufactured by Sigma, 84655) was added, and reaction was allowed to proceed at 37°C for 30 minutes to obtain a measurement sample. The absorbance of the measurement sample at 550 nm was measured using an iMark plate reader (manufactured by BioRad).

On the other hand, 190, 185, or 180 µL of sulfurous acid solution (manufactured by Fujifilm Wako Pure Chemical Corporation, 195-11955) was added to 10, 15, or 20 µL of plasma of the subject, and reaction was allowed to proceed at 37°C for 30 minutes, followed by measuring the absorbance as the background in the same manner as described above.

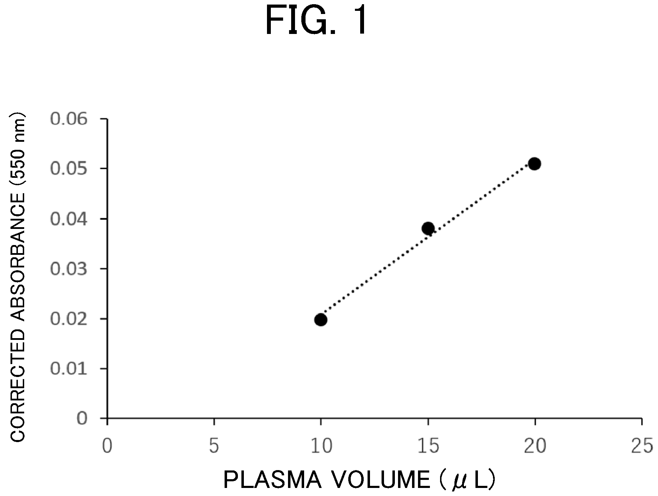

FIG. 1 illustrates the relationship between the absorbance of each measurement sample (corrected absorbance) obtained by subtracting the background value, and the plasma volume in the sample. The corrected absorbance was found to have increased in proportion to the plasma volume.

### [Comparative Example: Measurement of Plasmalogen in Human Plasma by LC-MS]

### 1. Preparation of Stock Standard Solutions

As reagents, phosphatidylethanolamine (PE, manufactured by Funakoshi Co., Ltd., A-34, dissolved in chloroform at 10 mg/mL), phosphatidylcholine (PC, manufactured by Funakoshi Co., Ltd., A-29B, dissolved in chloroform at 5 mg/mL), and sphingomyelin (SM, manufactured by Funakoshi Co., Ltd., A-401) were used. Using chloroform, 5 mg/mL SM was prepared from 25 mg of SM.

In separate screw Eppendorf tubes, 100 µL of PE and 200 µL each of PC and SM were placed. The reagents were dried under N₂ gas, and 1 mL of isopropanol/chloroform (2:1) was added to each of PE, PC, and SM, followed by adjusting the concentration to 1 mg/mL. After mixing, the resulting mixtures were subjected to ultrasonic treatment, and then stored as stock standard solutions at -30°C.

### 2. Preparation of Standard Solutions for Measurement

On ice, the stock standard solutions were diluted with HIP (3:2) (PE and PC were diluted to 5, 10, 15, 20, 25, or 50 µg/mL, and SM was diluted to 20, 40, 60, 80, 100, or 200 µg/mL), and each dilution was passed through a 0.45-µm filter to obtain a standard solution.

### 3. Lipid Extraction from Blood

Venous blood of the same subject as in the Test Example was collected into a blood collection tube containing heparin (manufactured by Terumo Corporation), centrifuged at 1000×g for 5 minutes, and plasma as the supernatant was collected and cryopreserved. The stored plasma was thawed by immersion in water at room temperature, and centrifuged at 13,000 rpm for 5 minutes. The plasma was subjected to pipetting with a 1-mL pipette, and then 280 µL of the plasma was transferred to an Eppendorf tube. A PLA1 (×2) solution was prepared as needed by mixing phospholipase A1 (manufactured by Sigma) and 0.1 M citrate buffer (HCl, pH 4.5) at 1:1, and 70 µL of the PLA1 (×2) solution was added to the above Eppendorf tube, followed by mixing. The resulting mixture was allowed to react at 45°C for 1 hour, and immediately thereafter, the tube was immersed in ice water to stop the reaction. After mixing, the PLA1-treated plasma was subjected to pipetting and aliquoted in 100-µL volumes into Eppendorf tubes.

Lipids were extracted by the hexane-isopropanol (HIP) method as follows. To the plasma, 800 µL of HIP (3:2) was added, followed by mixing. The resulting mixture was subjected to ultrasonic treatment for 5 minutes, and 400 µL of an aqueous Na₂SO₄ solution (1 g/15 mL) was added thereto, followed by mixing. The resulting mixture was left to stand for 5 minutes. Thereafter, 400 µL of the upper layer was taken without pipetting, and transferred to another Eppendorf tube. Further, 400 µL of HIP (7:2) was added, followed by mixing. After leaving the resulting mixture to stand for 5 minutes, 300 µL of the upper layer was taken without pipetting, and combined with the upper layer that had been collected in the Eppendorf tube, to obtain a sample. The sample was stored at -30°C until measurement.

### 4. LC-MS

Before measurement, the sample was dried under N₂ gas. The sample was redissolved in 200 µL of HIP (3:2) and passed through a 0.45-µm filter.

The standard solutions and the sample prepared were each placed in a legged glass insert, and 10 µL of each standard solution or sample was injected into LC-MS. The conditions for LC-MS were as follows.
Column: CHEMCOBOND 5OH (Diol) 3.0 × 30, 30 × 250 (w) (manufactured by Chemco Plus Co., Ltd.)
Column temperature: 50°C
Ionization mode: API-ES
Polarity: negative
Scan mass range low: 680
Scan mass range high: 950
Gas temperature: 300°C
Drying gas flow: 10.0 L/min
Nebulizer Pressure: 45 psig
Vcap (negative): 3500 V
Solvent A: 53 mM formic acid-acetonitrile
Solvent B: 60 mM ammonium formate and 53 mM formic acid-water

Table 1 illustrates the time schedule of LC-MS.

**[Table 1]**

| Time (minutes) | Solvent A (%) | Solvent B (%) | Flow rate (mL/min) |
|---|---|---|---|
| 2:00 | 90.0 | 10.0 | 0.5 |
| 15:00 | 70.0 | 30.0 | 0.5 |
| 17:00 | 70.0 | 30.0 | 0.5 |
| 17:50 | 90.0 | 10.0 | 0.5 |
| 25:00 | 90.0 | 10.0 | 0.5 |

5. Results

In Comparative Example, the concentration of PL-PE in plasma as quantified by LC-MS was 0.053 µg/µL.

[Example 1: Measurement of Plasmalogen in Human Plasma Based on Absorbance Using Schiff Reagent]

### 1. Preparation of sPls

Plasmalogen (sPls) consisting only of PL-PE was prepared from scallop (*Mizuhopecten yessoensis*) by the following method. sPls was extracted from raw scallop mantle by the method described in Japanese Patent No. 7304643, which uses hexane for the extraction. The steps of extracting plasmalogen from the scallop were as follows.
(1) Kokulase P (manufactured by Mitsubishi-Chemical Foods Corporation) and phospholipase A1 (PLA1, manufactured by Mitsubishi-Chemical Foods Corporation) were added to the scallop, followed by allowing degradation to proceed for 1 hour.
(2) After addition of hexane/isopropanol, the resulting mixture was stirred and then left to stand, followed by suction filtration of the supernatant.
(3) An aqueous sodium sulfate solution was added to the filtrate, followed by mixing well.
(4) The upper layer was evaporated to dryness with a rotary evaporator.
(5) To the dried product, 40 mL of acetone cooled to 4°C was added, followed by mixing.
(6) Separation was performed using a centrifuge at 3000 rpm for 10 minutes under conditions at 4°C.
(7) The supernatant was discarded, and the precipitate was collected.
(8) The collected precipitate was dried overnight in a desiccator.

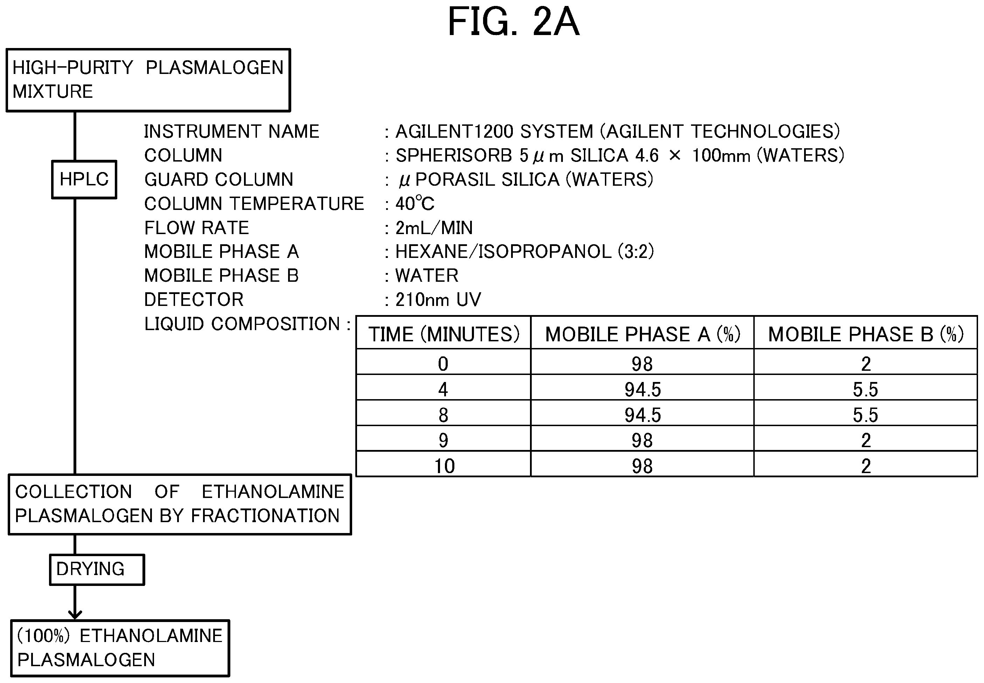

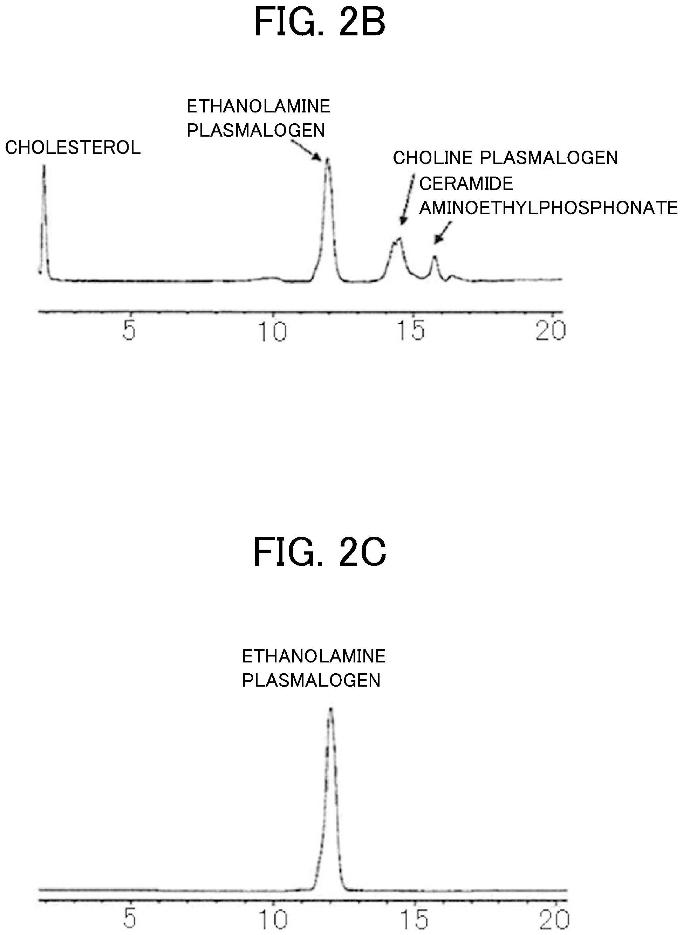

In accordance with Japanese Patent No. 5430566, separation by HPLC was performed using the mobile-phase solvents listed in FIG. 2A. FIG. 2B illustrates the lipid composition in the fraction extracted by the Steps (1) to (8) above. As illustrated in FIG. 2C, only PL-PE was collected by fractionation and used as sPls.

### 2. Preparation of Calibration Curve

sPls was dissolved in ethanol to a concentration of 0.005, 0.01, 0.05, or 0.1 µg/µL, to obtain an sPls solution. To 15 µL of the sPls solution at each concentration, 185 µL of a Schiff solution (pH<2.0, manufactured by Sigma, 84655) was added, and reaction was allowed to proceed at 37°C for 30 minutes, followed by measuring the absorbance at 550 nm in the same manner as in Test Example 1 above.

### 3. Measurement of Plasmalogen in Human Plasma

To 15 µL of plasma prepared from venous blood of the same subject as in Test Example 1, 185 µL of Schiff reagent was added, and reaction was allowed to proceed at 37°C for 30 minutes to obtain a measurement sample. The absorbance at 550 nm of the measurement sample was measured in the same manner as in Test Example 1 above.

On the other hand, 185 µL of sulfurous acid solution was added to 15 µL of plasma of the above subject, and reaction was allowed to proceed at 37°C for 30 minutes, followed by measuring the absorbance as the background in the same manner as in Test Example 1 above.

### 4. Results

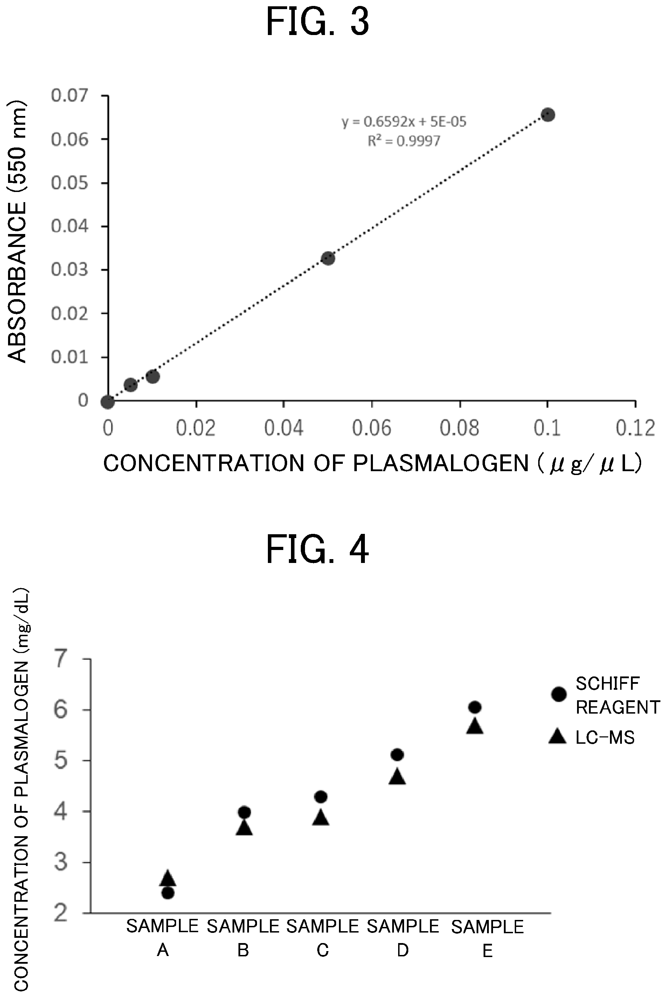

The prepared calibration curve is presented in FIG. 3. A favorable positive correlation was observed between the concentration of sPls and the absorbance (correlation coefficient = 0.9997).

The absorbance (corrected absorbance) of the measurement sample obtained by subtracting the background value was 0.038, and the concentration of plasmalogen in the plasma as calculated from the calibration curve was 0.058 µg/µL. Based on comparison with the result of Comparative Example, the above value was greater than the concentration of PL-PE, 0.053 µg/µL. This is because the concentration of plasmalogen calculated from the calibration curve also includes the concentration of choline plasmalogen in plasma.

### [Test Example 2: Study of Fluorescence of Schiff Reagent in Reaction with Aldehyde Derived from Plasmalogen]

Since fuchsin contained in Schiff reagent has a structure having a plurality of benzene rings, the possibility that fuchsin emits fluorescence was studied. HeLa cells that synthesize plasmalogen were fixed with a paraformaldehyde solution and then reacted with Schiff reagent at 37°C for 30 minutes. Fluorescence of the obtained sample was detected with white laser of a microscope SP8 (manufactured by Leica).

### (Results)

The sample was found to have a maximum excitation wavelength of 580 nm and a maximum fluorescence wavelength of 620 nm. Since fluorescence was not detected in plasmalogen synthesis-deficient cells subjected to the same treatment, fuchsin that had reacted with an aldehyde derived from plasmalogen was determined to have fluorescence.

### [Example 2: Measurement of Plasmalogen in Human Plasma Based on Fluorescence Using Schiff Reagent]

In general, measurement using fluorescence intensity has higher sensitivity than measurement using absorbance. Therefore, measurement of plasmalogen in plasma based on fluorescence was attempted by the same method as in Example 1 above except that fluorescence intensity was measured instead of absorbance. For the measurement of the fluorescence intensity, a fluorescence measuring apparatus DTX880 (manufactured by Beckman Coulter) was used. The wavelength of the excitation light was set to 535 nm using an excitation filter, and fluorescence at 595 nm was detected using a fluorescence filter. In the same manner as in Example 1 above, the present Example used absorbance to measure plasmalogen in plasma.

### (Results)

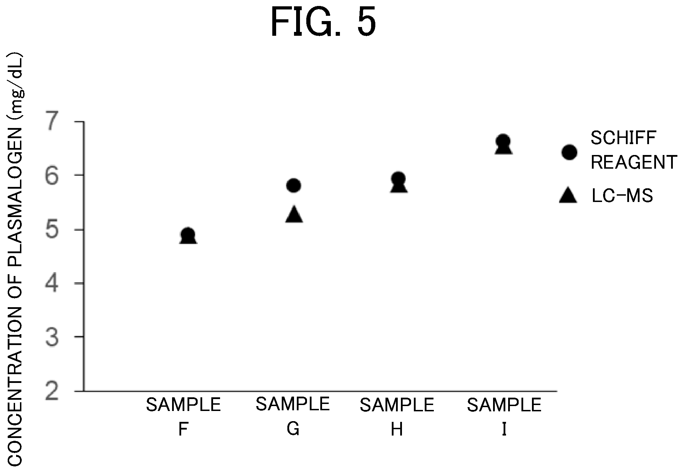

FIG. 4 illustrates the plasmalogen concentrations of measurement samples A to E as measured using fluorescence intensity or LC-MS. FIG. 5 illustrates the plasmalogen concentrations of measurement samples F to I as measured using absorbance or LC-MS. Also, when the fluorescence intensity was used, plasmalogen in plasma could be quantified.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2024-000431, filed on January 5, 2024, the entire disclosure of which is incorporated by reference herein.

### Industrial Applicability

The present disclosure is useful for quantification of plasmalogen.

## Claims

1. A method of quantifying plasmalogen, the method comprising:
a measurement sample preparation step of mixing a specimen containing protein and plasmalogen with Schiff reagent, to obtain a measurement sample;
a control sample preparation step of mixing the specimen with a solvent of the Schiff reagent, to obtain a control sample;
a measurement step of measuring a degree of a first optical property of the measurement sample after reaction of the Schiff reagent with an aldehyde generated from a vinyl ether bond of the plasmalogen, and a degree of an optical property of the control sample; and
a determination step of determining a plasmalogen concentration in the specimen by comparing a corrected degree obtained by correcting the degree of the first optical property with the degree of the optical property of the control sample, with a degree of a second optical property of a standard sample containing Schiff reagent and plasmalogen having a known concentration, the degree of the second optical property being obtained after reaction of the Schiff reagent with an aldehyde generated from a vinyl ether bond of the plasmalogen.

2. The method of quantifying plasmalogen according to claim 1, wherein
the standard sample is a plurality of standard samples containing the plasmalogen at concentrations different from each other and the Schiff reagent, and
in the determination step, the plasmalogen concentration in the specimen is determined based on a calibration curve prepared from the degrees of the second optical property of the plurality of standard samples after the reaction, and on the corrected degree.

3. The method of quantifying plasmalogen according to claim 1 or 2, wherein the degree of the first optical property, the degree of the optical property of the control sample, and the degree of the second optical property are measured as fluorescence intensities.

4. The method of quantifying plasmalogen according to claim 1 or 2, wherein the optical properties are color development.

5. The method of quantifying plasmalogen according to claim 1 or 2, wherein the degree of the first optical property, the degree of the optical property of the control sample, and the degree of the second optical property are measured as absorbances.

6. The method of quantifying plasmalogen according to claim 1 or 2, wherein the plasmalogen having a known concentration is dissolved in ethanol.

7. The method of quantifying plasmalogen according to claim 1 or 2, wherein the specimen is cell-free.

8. The method of quantifying plasmalogen according to claim 1 or 2, wherein the solvent of the Schiff reagent is a sulfurous acid solution.

9. A kit for quantifying plasmalogen, comprising:
Schiff reagent; and
a solvent of the Schiff reagent.
